(19) Europäisches Patentamt
European Patent Office
Office européen des brevets

(11) Publication number: **0 065 814**
**B1**

(12) **EUROPEAN PATENT SPECIFICATION**

(45) Date of publication of patent specification: **14.08.85**

(51) Int. Cl.⁴: **C 07 C 33/46,** C 07 C 29/40

(21) Application number: **82301966.6**

(22) Date of filing: **16.04.82**

(54) **1,1-Bis phenylalkan-1-ols and processes for their preparation.**

(30) Priority: **12.05.81 GB 8114394**

(43) Date of publication of application:
**01.12.82 Bulletin 82/48**

(45) Publication of the grant of the patent:
**14.08.85 Bulletin 85/33**

(84) Designated Contracting States:
**BE CH DE FR GB IT LI NL**

(56) References cited:
**DE-C-1 076 112**
**GB-A-1 047 564**
**US-A-2 921 940**

**Norman L. Allinger et al. Organic Chemistry 1971, p. 316-317**
**Chemical Abstracts 77 (1972) 151318 a;**
**J.Org.Chem.(1968) 33 (7), 2852-7.**

(73) Proprietor: **IMPERIAL CHEMICAL INDUSTRIES PLC**
**Imperial Chemical House Millbank**
**London SW1P 3JF (GB)**

(72) Inventor: **Jones, Raymond Vincent Heavon**
**26 Claerendon Road**
**Linlithgow (GB)**
Inventor: **Fleming, Ian George Cameron**
**56 Newmains Road**
**Kirkliston Scotland (GB)**

(74) Representative: **Houghton, Malcolm John et al**
**Imperial Chemical Industries PLC Legal Department: Patents PO Box 6**
**Welwyn Garden City Herts, AL7 1HD (GB)**

**EP 0 065 814 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention relates to tertiary alcohols, more particularly to 1,1-bis-phenyl-alkan-1-ols, and to processes for their preparation.

The compound 1,1-bis(p-chlorophenyl)-ethanol and its p-fluoro analogue are known from Chem.Abs [1951] 8190(f), Chem.Abs. [1952] 6626(a), and JACS *72* [1950] 2279—80 as synergists for DDT against houseflies. The compound 1-(o-chlorophenyl)-1-(p-chlorophenyl)-2-methyl-1-propanol is known from Chem.Abs. *58* 5568(g) as a precursor of the corresponding propene which is used for testing in treatment of metastatic adrenal carcinoma. The synthesis of the compound 1-(o-chlorophenyl)-1-(p-chlorophenyl)-1-propanol, used to substantiate the structure of a N-nitrosoketimine reaction product, is described in J. Org Chem [1968], *33(7)*, 2852—7.

According to the present invention there are provided 1,1-bis-phenyl-alkan-1-ols of the general formula (I):

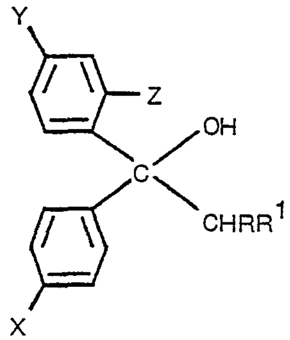

(I)

wherein R and $R^1$, which may be the same or different, are hydrogen or alkyl, preferably $C_1$—$C_4$ alkyl, X and Z are each independently chlorine or fluorine, and Y is hydrogen, chlorine or fluorine provided that when R and $R^1$ are both methyl or one of R and $R^1$ is hydrogen and the other is methyl and Y is hydrogen, X and Z are not both chlorine. It is preferred that R and $R^1$ are both hydrogen.

According to a further feature of the invention there is provided a process for the preparation of the compounds of formula (I) which comprises reacting an alkyl magnesium halide of formula (II):

$$RR^1HCMg.Hal \qquad (II)$$

with a benzophenone of formula (III):

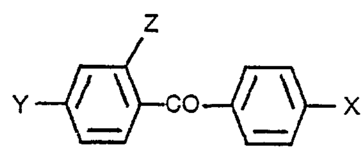

(III)

wherein R, $R^1$, X, Y and Z have the previously defined meanings and Hal is chlorine, bromine or iodine.

This process is carried out by methods which are well known in the art for the preparation and reaction of Grignard reagents, of which the alkyl magnesium halides of formula (II) are examples, and ketones such as the benzophenones of formula (III).

Thus, the alkyl magnesium halide of formula (II) may be prepared in known manner by reaction of an alkyl halide of formula (IV):

$$RR^1HC.Hal. \qquad (IV)$$

wherein R, $R^1$ and Hal. have the previously defined meanings, with metallic magnesium in an anhydrous ether solvent, for example, diethyl ether or tetrahydrofuran, optionally in admixture with an inert organic solvent such as toluene, and generally with the application of gentle heat to assist completion of the reaction. The benzophenone of formula (III) is then added, preferably as a solution in an inert organic solvent such as toluene, to the solution of the Grignard reagent, the reaction temperature being kept at or slightly above normal room temperature, for example, at 25—30°C.

The tertiary alcohol of formula (I) may then be isolated from the reaction mixture in known manner, for example, by pouring the mixture into dilute aqueous ammonium chloride solution and separating the organic layer, which is then dried, followed by removal of the solvent by evaporation or distillation to leave the product.

The benzophenones of formula (III) may be obtained by Friedel-Crafts reaction between a benzoyl halide of formula (V):

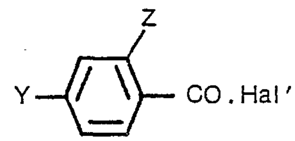

(V)

and a benzene derivative of formula (VI):

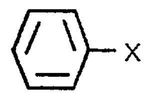

(VI)

wherein X, Y and Z have the previously defined meanings and Hal' represents bromine or preferably chlorine, in the presence of a Lewis acid such as aluminium chloride.

Benzophenones of formula (III) in which X and Y are both fluorine may also be obtained from a diphenylmethane of formula

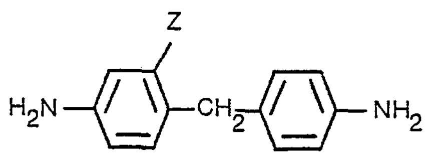

wherein Z has the previously defined meaning, by the method described in European Patent Publication No. 4710.

According to a yet further feature of the invention there is provided an alternative process for the preparation of the compounds of formula (I) which comprises reacting a compound of formula (VII):

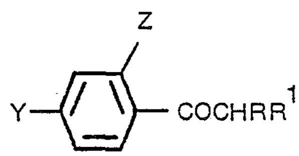

(VII)

with a Grignard compound of formula (VIII)

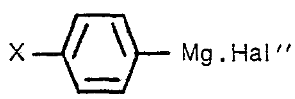

(VIII)

wherein R, $R^1$, X, Y and Z have the previously defined meanings and Hal'' is bromine or iodine.

This latter process is carried out essentially as described for the previous process.

The tertiary alcohols of formula (I) are useful intermediates for fungicides. Those in which $R = R^1 = H$ are intermediates for the preparation of fungicidal compounds of formula (X):

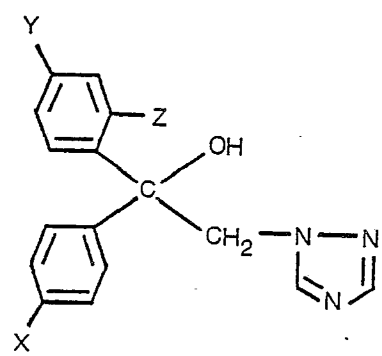

(X)

wherein X, Y and Z have the previously defined meanings. Compounds of formula (X) and methods for their preparation are disclosed in European Patent Application No. 80300693.1 (Publication No. 0015756) and in co-pending European Patent Application No. 82301964.1 (Publication No. 0065354).

The tertiary alcohols of formula (I) in which $R = R^1 = H$ may be converted into compounds of formula

(X), by for example, reaction of the tertiary alcohols themselves or their alkene dehydration products with chlorine, bromine, hypochlorous acid or hypobromous acid in aqueous medium to give the corresponding halohydrin of formula (XII):

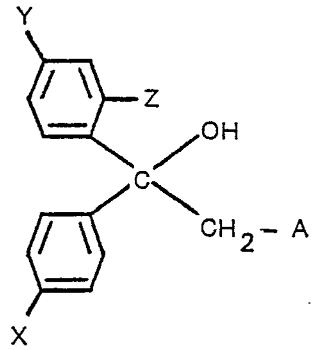

(XII)

wherein X, Y and Z have the previously defined meanings and A is chlorine or bromine. The halohydrin is subsequently reacted with 1,2,4-triazole to give the compound of formula (X) via the epoxide of formula (XIII):

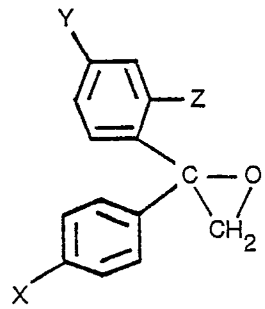

(XIII)

which is readily formed under the basic conditions of the triazole addition.

The alkene dehydration products referred to have the formula (XI):

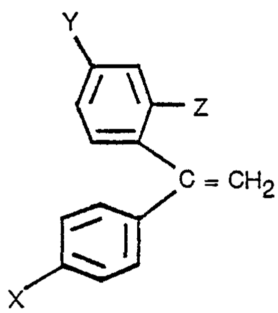

(XI)

and are obtained by dehydrating the tertiary alcohols by, for example, heating the alcohols in the presence of a catalytic amount of a strong acid such as *p*-toluenesulphonic acid.

Particularly useful tertiary alcohols of formula (I) for the synthesis of compounds of formula (X) are those having R = $R^1$ = H and the following substitution patterns for X, Y and Z:

| X | Y | Z |
|---|---|---|
| F | H | F |
| F | H | Cl |
| Cl | H | Cl |
| F | Cl | Cl |

The invention is illustrated by the following Examples in which parts and percentages are by weight.

Example 1

*1-(2-chlorophenyl)-1-(4-fluorophenyl)ethanol*

A solution of methyl bromide (89 parts) in toluene (43 parts) is added, over about 30 minutes, to a stirred mixture of magnesium turnings (20 parts), toluene (130 parts) and tetrahydrofuran (122 parts), maintaining the temperature at 30—40°C. After the magnesium is dissolved, 2-chloro-4'-fluorobenzophenone (130 parts) in toluene (125 parts) is added, whilst maintaining the temperature at 25—30°C. The mixture is drowned out into 5% aqueous ammonium chloride (1500 parts) and stirred for 30 minutes. The organic layer is separated, dried over anhydrous sodium sulphate and the solvent removed, under vacuum, at less than 40°C, to leave a brown oil (131 parts) containing 90% (by GLC, 10% SE30 at 200°C) of 1-(2-chlorophenyl)-1-(4-fluorophenyl)ethanol (85% conversion based on 2-chloro-4'-fluorobenzophenone). Distillation under vacuum, from sodium carbonate, gives 1-(2-chlorophenyl)-1-(4-fluorophenyl)ethanol contaminated with a small amount of 1-(2-chlorophenyl)-1-(4-fluorophenyl)ethene. Elution through silica gel, with toluene, removes the latter to leave a colourless, viscous oil which is pure *1-(2-chlorophenyl)-1-(4-fluorophenyl)ethanol* (b.p. 117.5°/0.15 mm Hg; with slight decomposition).

*P.m.r. spectrum:* ($CDCl_3$/TMS): 1.92 (3H: singlet); 3.27 (1H: labile singlet); 6.75—7.85 (8H: aromatic complex).

The 2-chloro-4'-fluorobenzophenone used in this Example was obtained by a Friedel-Craft reaction between fluorobenzene and 2-chlorobenzoyl chloride using an aluminium chloride catalyst.

Example 2

*1-(2-fluorophenyl)-1-(4-fluorophenyl)ethanol*

The procedure described in Example 1 was repeated except that the 130 parts of 2-chloro-4'-fluorobenzophenone were replaced by 163 parts of 2,4'-difluorobenzophenone, which was converted into the title compound in 85% yield.

Purification, as described in Example 1 leaves a colourless liquid which is pure *1-(2-fluorophenyl)-1-(4-fluorophenyl)ethanol* (b.p. 102°/0.1 mm Hg; with slight decomposition).

*P.m.r. spectrum:* ($CDCl_3$/TMS): 1.90 (3H: singlet); 2.7 (1H: labile singlet); 6.75—7.75 (8H: aromatic complex).

The 2,4'-difluorobenzophenone used in this Example was obtained by a Friedel-Crafts reaction between fluorobenzene and 2-fluorobenzoyl chloride using an aluminium chloride catalyst.

**Claims**

1. A compound of the formula (I):

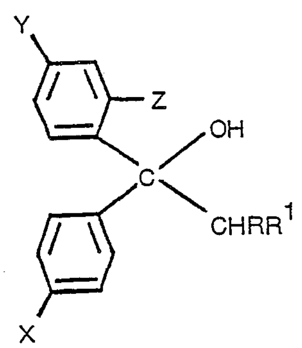

(I)

wherein R and $R^1$, which may be the same or different, are hydrogen or alkyl, X and Z are each independently chlorine or fluorine, and Y is hydrogen, chlorine or fluorine provided that when R and $R^1$ are both methyl or one of R and $R^1$ is hydrogen and the other is methyl and Y is hydrogen, X and Z are not both chlorine.

2. A compound according to claim 1 in which R and $R^1$ are both hydrogen.

3. 1-(2-chlorophenyl)-1-(4-fluorophenyl)ethanol.

4. 1-(2-chlorophenyl)-1-(4-chlorophenyl)ethanol.

5. 1-(2-fluorophenyl)-1-(4-fluorophenyl)ethanol.

6. 1-(2,4-dichlorophenyl)-1-(4-fluorophenyl)ethanol.

7. A process for the preparation of the compounds of formula (I) in claim 1 which comprises reacting an alkyl magnesium halide of formula (II):

$$RR^1HCMg.Hal \qquad (II)$$

with a benzophenone of formula (III):

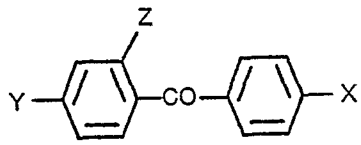

(III)

wherein R, $R^1$, X, Y and Z have the meanings given in claim 1 and Hal is chlorine, bromine or iodine.

8. A process for the preparation of the compounds of formula (I) in claim 1 which comprises reacting a compound of formula (VII):

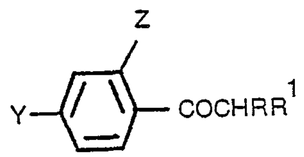

(VII)

with a Grignard compound of formula (VIII):

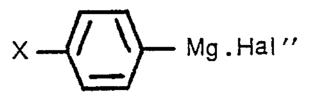

(VIII)

wherein R, $R^1$, X, Y and Z have the meanings given in claim 1 and Hal'' is bromine or iodine.

**Patentansprüche**

1. Verbindung der Formel (I)

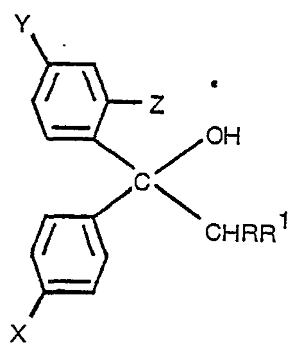

(I)

worin R und $R^1$, welche gleich oder verschieden sein können, für Wasserstoff oder Alkyl stehen, X und Z jeweils unabhängig für Chlor oder Fluor stehen und Y für Wasserstoff, Chlor oder Fluor steht, mit der Maßgabe, daß, wenn R und $R^1$ beide für Methyl stehen oder eines der Symbole R und $R^1$ für Wasserstoff und das andere für Methyl steht und Y für Wasserstoff steht, X und Z nicht beide für Chlor stehen.

2. Verbindung nach Anspruch 1, bei welcher R und $R^1$ beide für Wasserstoff stehen.

3. 1-(2-Chlorophenyl)-1-(4-fluorophenyl)ethanol.

4. 1-(2-Chlorophenyl)-1-(4-chlorophenyl)ethanol.

5. 1-(2-Fluorophenyl)-1-(4-fluorophenyl)ethanol.

6. 1-(2,4-Dichlorophenyl)-1-(4-fluorophenyl)ethanol.

7. Verfahren zur Herstellung der Verbindung der Formel (I) von Anspruch 1, bei welchem ein Alkylmagnesiumhalogenid der Formel (II)

$$RR^1HCMg.Hal \qquad (II)$$

mit einem Benzophenon der Formel (III)

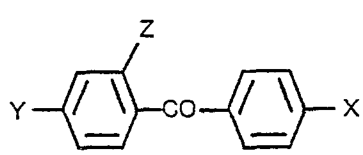

(III)

umgesetzt wird, wobei R, $R^1$, X, Y und Z die in Anspruch 1 angegebenen Bedeutungen besitzen und Hal für Chlor, Brom oder Jod steht.

8. Verfahren zur Herstellung der Verbindungen der Formel (I) von Anspruch 1, bei welchem eine Verbindung der Formel (VII)

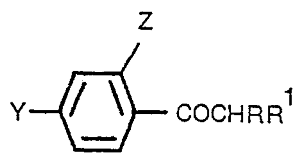

(VII)

mit einer Grignard-Verbindung der Formel (VIII)

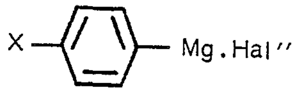

(VIII)

umgesetzt wird, wobei R, $R^1$, X, Y und Z die in Anspruch 1 angegebenen Bedeutungen besitzen und Hal'' für Brom oder Jod steht.

**Revendications**

1. Composé de formule (I):

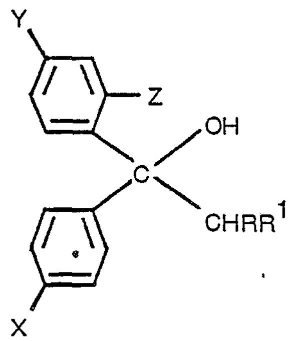

(I)

où R et $R^1$, qui peuvent être identiques ou différents, représentent des atomes d'hydrogène ou radicaux alcoyle, X et Z représentent chacun indépendamment un atome de chlor ou de fluor, et Y représente un atome d'hydrogène, de chlore ou de fluor, avec la restriction que lorsque R et $R^1$ représentent tous deux des radicaux méthyle ou l'un d'entre R et $R^1$ représente un atome d'hydrogène et l'autre représente un radical méthyle et Y représente un atome d'hydrogène, X et Z ne représentent pas tous deux des atomes de chlore.

2. Composé suivant la revendication 1, dans lequel R et $R^1$ représentent tous deux des atomes d'hydrogène.

3. Le 1-(2-chlorophényl)-1-(4-fluorophényl)éthanol.

4. Le 1-(2-chlorophényl)-1-(4-chlorophényl)éthanol.

5. Le 1-(2-fluorophényl)-1-(4-fluorophényl)éthanol.

6. Le 1-(2,4-dichlorophényl)-1-(4-fluorophényl)éthanol.

7. Procédé de préparation des composés de formule (I) suivant la revendication 1, qui comprend la réaction d'un halogénure d'alcoylmagnésium de formule (II):

$$RR^1HCMg.Hal \quad (II)$$

avec une benzophénone de formule (III):

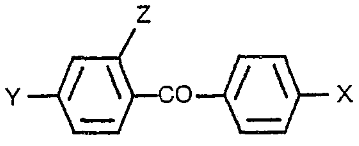

(III)

où R, $R^1$, X, Y et Z ont les significations données dans la revendication 1 et Hal représente un atome de chlore, de brome ou d'iode.

8. Procédé de préparation des composés de formule (I) suivant la revendication 1, qui comprend la réaction d'un composé de formule (VII):

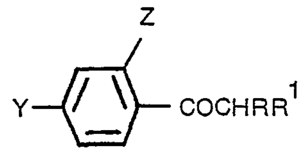

(VII)

avec un composé de Grignard de formule (VIII):

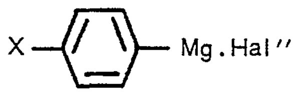

(VIII)

où R, R', X, Y et Z ont les significations données dans la revendication 1 et Hal'' représente un atome de brome ou d'iode.